(19) Europäisches Patentamt
European Patent Office
Office européen des brevets

(11) Veröffentlichungsnummer **0 019 060**
**B1**

(12) # EUROPÄISCHE PATENTSCHRIFT

(45) Veröffentlichungstag der Patentschrift: 21.04.82

(21) Anmeldenummer: 80101582.7

(22) Anmeldetag: 26.03.80

(51) Int. Cl.³: **C 07 C 49/782**, C 07 C 49/792, C 07 C 49/813, C 07 C 49/84, C 07 C 45/45, C 09 K 3/34, G 02 F 1/13

(54) **Diketone, Verfahren zu ihrer Herstellung und ihre Verwendung als Komponenten flüssigkristalliner Dielektrika.**

(30) Priorität: 10.05.79 DE 2918775

(43) Veröffentlichungstag der Anmeldung: 26.11.80 Patentblatt 80/24

(45) Bekanntmachung des Hinweises auf die Patenterteilung: 21.04.82 Patentblatt 82/16

(84) Benannte Vertragsstaaten: AT BE CH DE FR GB IT NL SE

(56) Entgegenhaltungen:
DE-A1-2 337 396
DE-A1-2 451 006
DE-A1-2 636 684
FR-A-2 165 836

(73) Patentinhaber: **Merck Patent Gesellschaft mit beschränkter Haftung, Frankfurter Strasse 250, D-6100 Darmstadt (DE)**

(72) Erfinder: **Eidenschink, Rudolf, Dr., Dessauer Strasse 57, D-6110 Dieburg (DE)**
Erfinder: **Pohl, Ludwig, Dr., Hoffmannstrasse 54, D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 019 060 B1

# Diketone, Verfahren zu ihrer Herstellung und ihre Verwendung als Komponenten flüssigkristalliner Dielektrika

Für Flüssigkristall-Anzeigeelemente werden die Eigenschaften nematischer oder nematisch-cholesterischer flüssigkristalliner Materialien ausgenutzt, ihre optischen Eigenschaften wie Lichtdurchlässigkeit, Lichtstreuung, Doppelbrechung, Reflexionsvermögen oder Farbe unter dem Einfluß elektrischer Felder signifikant zu verändern. Die Funktion derartiger Anzeigeelemente beruht dabei beispielsweise auf den Phänomenen der dynamischen Streuung, der Deformation aufgerichteter Phasen, dem Schadt-Helfrich-Effekt in der verdrillten Zelle oder dem nematisch-cholesterischen Phasenübergang.

Für die technische Anwendung dieser Effekte in Flüssigkristall-Anzeigeelementen werden flüssigkristalline Materialien benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrische Gleich- und Wechselfelder. Ferner wird von technisch verwendbaren flüssigkristallinen Materialien eine flüssigkristalline Mesophase im Temperaturbereich von mindestens 0°C bis +50°C, bevorzugt von −10°C bis +70°C, und eine Viskosität bei Raumtemperatur von nicht mehr als 60 cP gefordert. Schließlich dürfen sie im Bereich des sichtbaren Lichts keine Eigenabsorption aufweisen, d. h., sie müssen farblos sein.

Es ist bereits eine Anzahl von flüssigkristallinen Verbindungen bekannt, die den an Dielektrika für elektronische Bauelemente gestellten Stabilitätsanforderungen genügen und auch farblos sind. Hierzu gehören insbesondere die in der DE-OS 2 139 628 beschriebenen p,p'-disubstituierten Benzoesäurephenylester, die in der DE-OS 2 356 085 beschriebenen p,p'-disubstituierten Biphenylderivate oder die in der DE-OS 2 636 684 beschriebenen Phenylcyclohexanderivate.

Diese und auch andere bisher in der Praxis verwendete flüssigkristalline Substanzklassen sind aus miteinander direkt oder über eine Brückengruppe verbundenen aromatischen oder hydroaromatischen Ringen mit endständigen Alkyl- und/oder Alkoxygruppen aufgebaut. Diese Struktur gewährleistet die für das Auftreten einer flüssigkristallinen, insbesondere nematischen Mesophase notwendige Starrheit des Moleküls. Viele dieser Verbindungen besitzen jedoch einen verhältnismäßig wenig polaren Charakter und sind daher oft nicht dazu geeignet, polare Dotierungsmittel und/oder dichroitische Farbstoffe in solchen Konzentrationen zu lösen, wie es für manche Anwendungsarten erwünscht ist.

Der Erfindung liegt daher die Aufgabe zugrunde, flüssigkristalline Verbindungen mit polaren Charakter zur Verfügung zu stellen, in denen auch stark polare Dotierungsmittel und/oder dichroitische Farbstoffe in ausreichendem Maße löslich sind, um Farbschalter auf der Basis des guest-host-Effekts herstellen zu können.

Es wurde nun überraschenderweise gefunden, daß die Diketone der Formel (I)

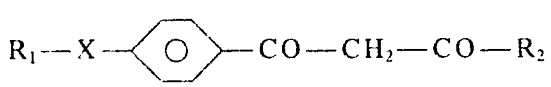

(I)

worin

$R_1$ Alkyl, Perfluoralkyl oder Alkoxy mit 1—18 C-Atomen,
$R_2$ Alkyl oder Perfluoralkyl mit 1—12 C-Atomen und
X 1,4-trans-Cyclohexylen, 1,4-Phenylen oder 4,4'-Cyclohexylphenyl
bedeutet,

hervorragend als Komponenten polarer flüssigkristalliner Dielektrika geeignet sind. Die Verbindungen der Formel (I) besitzen flüssigkristalline Mesophasen und sind aufgrund ihres polaren Charakters fähig, stark polare Dotierungsmittel und/oder dichroitische Farbstoffe in bislang kaum erreichten Konzentrationen zu lösen. Weiterhin können die Verbindungen der Formel (I) mit Metallionen Salze bzw. Komplexverbindungen bilden, die als die notwendige elektrische Leitfähigkeit bewirkende Dotierungsmittel in flüssigkristallinen Dielektrika für die dynamische Streuung verwendet werden können. Schließlich besitzen sie eine hervorragende stabilisierende Wirkung auf solche flüssigkristallinen Verbindungen, die gegen Licht und UV-Bestrahlung empfindlich sind.

Gegenstand der Erfindung sind somit die Diketone der Formel (I), Verfahren zu ihrer Herstellung und ihre Verwendung als Komponenten flüssigkristalliner Dielektrika.

Gegenstand der Erfindung sind ferner flüssigkristalline Dielektrika mit einem Gehalt an mindestens einer Verbindung der Formel (I) sowie elektrooptische Anzeigeelemente auf der Basis einer Flüssigkristallzelle, die ein derartiges Dielektrikum enthalten.

Die erfindungsgemäßen Diketone sind entweder

4'-Cyclohexylacetophenonderivate der Formel (Ia)

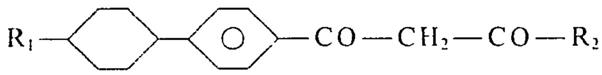

(Ia)

oder 4'-Phenylacetophenonderivate der Formel (Ib)

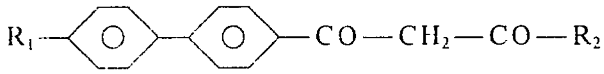

(Ib)

oder 4'-(4-Cyclohexylphenyl)-acetophenonderivate der Formel (Ic)

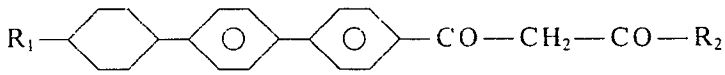

(Ic)

wobei die Reste $R_1$ und $R_2$ in den Teilformeln (Ia) bis (Ic) die bei der Formel (I) angegebene Bedeutung besitzen.

In den erfindungsgemäßen Verbindungen bedeutet der Rest $R_1$ eine Alkyl-, Alkoxy- oder Perfluoralkylgruppe mit 1 bis 18 Kohlenstoffatomen, die geradkettig oder verzweigt sein kann. Von den geradkettigen Resten $R_1$, also Methyl, Äthyl, n-Propyl, n-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl oder n-Octadecyl bzw. den analogen Alkoxy- oder Perfluoralkylgruppen sind die mit bis zu 12 Kohlenstoffatomen bevorzugt. Gelegentlich sind jedoch auch Verbindungen der Formel (I) mit verzweigtem Substituenten $R_1$ von Bedeutung, da diese manchmal bessere Löslichkeitseigenschaften in den üblichen flüssigkristallinen Grundmischungen aufweisen. Solche nicht geradkettigen Substituenten $R_1$ enthalten vorzugsweise nicht mehr als eine Kettenverzweigung. Bevorzugte verzweigte Substituenten sind die, in denen die Kohlenstoffkette an dem der Gruppe X benachbarten Kohlenstoffatom oder insbesondere an einem der beiden nächsten Kohlenstoffatome verzweigt ist. Von Bedeutung sind unter diesen solche verzweigten Gruppen, in denen sich an einer längeren Kohlenstoffkette in 1-, 2- oder 3-Stellung eine Methyl- oder Äthylgruppe befindet, beispielsweise Isopropyl, 1-Methylpropyl, 2-Methylpropyl, 1-Methylbutyl, 2-Methylbutyl, 3-Methylbutyl, 1-Methylpentyl, 1-Äthylpentyl, 2-Methylpentyl, 1-Methylhexyl, 2-Äthylhexyl oder 1-Methylheptyl. Diese Auswahlkriterien gelten analog auch für die entsprechenden Alkoxygruppen $R_1$. Wenn $R_1$ eine Perfluoralkylgruppe ist, sind unter diesen die nicht mehr als 8 Kohlenstoffatome enthaltenden bevorzugt, und insbesondere die mit 1—5 Kohlenstoffatomen; besonders einfach sind davon die Verbindungen zugänglich, in denen $R_1$ eine Trifluormethylgruppe ist. Bei den Verbindungen der Teilformeln (Ia) und (Ic) sind diejenigen bevorzugt, in denen $R_1$ eine Alkylgruppe ist; weil die Synthese der entsprechenden Alkoxy- und Perfluoralkylderivate schwieriger ist, ist deren Verwendung in flüssigkristallinen Dielektrika weniger wirtschaftlich.

Der Rest $R_2$ bedeutet in den erfindungsgemäßen Verbindungen Alkyl oder Perfluoralkyl mit 1—12 Kohlenstoffatomen. Bevorzugt werden hiervon solche Verbindungen, in denen $R_2$ Alkyl mit 1—8 Kohlenstoffatomen, insbesondere mit 1—4 Kohlenstoffatomen ist. Von den analogen Perfluoralkylderivaten sind die bevorzugt, in den $R_2$ bis zu 6, vorzugsweise 1—4 Kohlenstoffatome enthält, insbesondere die Trifluormethylgruppe bedeutet.

Die erfindungsgemäßen Verbindungen werden in für derartige Substanzen üblicher Weise hergestellt. Vorzugsweise setzt man ein Acetophenon der Formel (II)

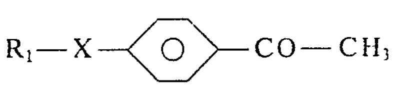

(II)

worin $R_1$ die in Formel (I) angegebene Bedeutung besitzt, in Gegenwart eines basischen Kondensationsmittels mit einem Carbonsäureester der Formel (III)

$$R_2—COO—Z \qquad (III)$$

worin Z eine niedere Alkylgruppe ist und $R_2$ die in Formel (I) angegebene Bedeutung besitzt, um, und hydrolysiert das zunächst entstandene Salz der Enol-Form des Diketons (I) zu diesem.

Die Acetophenone der Formel (II) sind zum Teil aus der Literatur bekannt, oder sie können analog zu den aus der Literatur bekannten Verbindungen dieser Art hergestellt werden, insbesondere durch Acetylierung einer Verbindung der Formel (IV)



(IV)

unter den Bedingungen einer Friedel-Crafts-Reaktion. In den Carbonsäureestern der Formel (III) bedeutet Z gewöhnlich eine Alkylgruppe mit bis zu 4 Kohlenstoffatomen, vorzugsweise Methyl oder Äthyl. Diese Ester sind aus der Literatur bekannt.

Die Umsetzung der Acetophenone der Formel (II) mit den Estern der Formel (III) wird unter den allgemein für derartige Claisen-Kondensationen üblichen Bedingungen durchgeführt, wobei als basische Kondensationsmittel insbesondere Natrium, Kalium, Natriumamid, Kaliumamid, Natriumhydrid oder Natrium- bzw. Kaliumalkoholate verwendet werden. Zweckmäßig werden die Umsetzungen in einem wasserfreien, inerten organischen Lösungsmittel ausgeführt, zum Beispiel Diäthyläther, Tetrahydrofuran, Benzol, Toluol, Xylol oder Petroläther. In den meisten Fällen entsteht bei dieser Umsetzung, die zweckmäßig bei einer Temperatur zwischen $-20\,°C$ und $+50\,°C$ durchgeführt wird, das Alkalisalz der Enolform des Diketons der Formel (I) als fester Niederschlag, der abfiltriert, mit reinem Lösungsmittel gewaschen und mit Wasser, ggf. in Gegenwart einer kleinen Menge einer Mineralsäure hydrolysiert wird. Wenn die Hydrolyse in Gegenwart eines mit Wasser nicht mischbaren organischen Lösungsmittels durchgeführt wird, wird die das Diketon (I) enthaltende organische Phase anschließend abgetrennt und eingedampft. Das zurückbleibende Diketon wird dann durch Umkristallisation aus einem geeigneten Lösungsmittel, zum Beispiel Methanol, Äthanol oder Essigsäureäthylester gereinigt. Weiterhin können die erfindungsgemäßen Diketone der Formel (I) hergestellt werden, indem man einen Benzoesäureester der Formel (V)

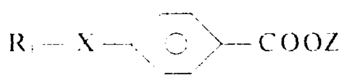

(V)

worin $R_1$ und Z die vorstehend angegebene Bedeutung besitzen, mit einem Keton der Formel (VI)

$$CH_3-CO-R_2 \qquad (VI)$$

unter Bedingungen umsetzt, die den für die Umsetzung der Acetophenone (II) mit den Carbonsäureestern (III) weitgehend analog sind.

Die Verbindungen der Formel (I) sind wertvolle Komponenten flüssigkristalliner Dielektrika, die zur Herstellung elektrooptischer Anzeigeelemente verwendet werden. Ihre Verwendbarkeit zu diesem Zweck ist wahrscheinlich auf das Vorliegen der tautomeren Enol-Strukturen (VIIa) und (VIIb)

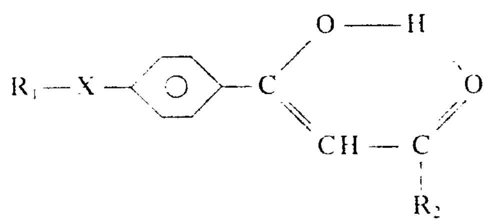

(VIIa)

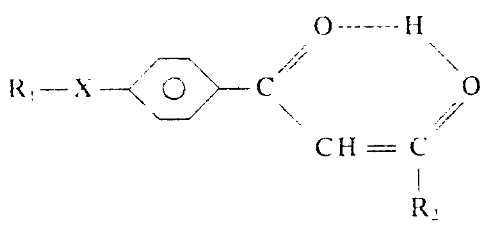

(VIIb)

zurückzuführen. Diese Annahme wird auch durch die Fähigkeit der erfindungsgemäßen Diketone zur Bildung von Komplexsalzen gestützt.

Die erfindungsgemäßen flüssigkristallinen Dielektrika bestehen aus zwei oder mehr Komponenten, darunter mindestens eine der Formel (I). Die weiteren Komponenten sind vorzugsweise nematische oder nematogene Substanzen aus den Klassen der Azobenzole, Azoxybenzole, Biphenyle, Schiffschen Basen, insbesondere Benzyliden-Derivate, Phenylbenzoate, Phenylcyclohexane, gegebenenfalls halogenierten Stilbene, Diphenylacetylen-Derivate, Diphenylnitrone und substituierten Zimtsäuren. Die wichtigsten als derartige weitere Komponenten in Frage kommenden Verbindungen lassen sich durch die Formel (VIII) charakterisieren:

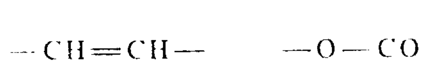

(VIII)

worin

A $-CH=CH-$ 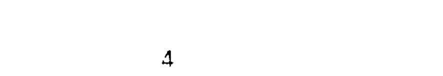

| | |
|---|---|
| $-CX'=CH-$ | 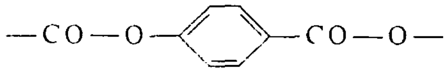 |
| $-CH=CX'-$ | 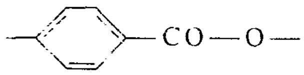 |
| $-C\equiv C-$ | 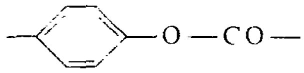 |
| $-N=N-$ | 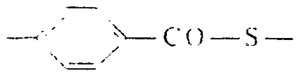 |
| $-N(O)=N-$ | 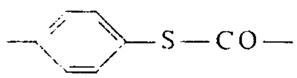 |
| $-N=N(O)-$ | $-CH=N-$ |
| $-O-CO-$ | $-N=CH-$ |
| $-CO-O-$ | $-CH=N(O)-$ |
| $-S-CO-$ | $-N(O)=CH-$ |
| $-CO-S-$ | oder eine C—C-Einfachbindung |

bedeutet. Weitere mögliche Komponenten der erfindungsgemäßen Dielektrika sind solche Verbindungen der Formel (VIII), in denen einer oder mehrere Phenylringe durch eine entsprechende Zahl von trans-Cyclohexylringen ersetzt sind;

X' bedeutet Halogen, vorzugsweise Cl. $R_3$ und $R_4$ sind gleich oder verschieden und können Alkyl-, Alkoxy-, Alkanoyl-, Alkanoyloxy- oder Alkoxycarbonyloxyreste mit bis zu 18, vorzugsweise bis zu 8 C-Atomen bedeuten; weiterhin kann einer dieser Reste auch eine Cyano-, Nitro- oder Isonitrilgruppe bedeuten. Bei den meisten dieser Verbindungen sind $R_3$ und $R_4$ vorzugsweise verschieden, wobei einer der Reste meist eine Alkyl- oder Alkoxygruppe bedeutet. Aber auch eine große Zahl anderer Varianten der vorgesehenen Substituenten sind gebräuchlich. Viele solcher Substanzen sind im Handel erhältlich.

Die erfindungsgemäßen Dielektrika enthalten in der Regel mindestens 30, vorzugsweise 50—99, insbesondere 60—98 Gewichtsteile der Verbindungen der Formeln (I) und gegebenenfalls (VIII). Hiervon entfallen bevorzugt mindestens 15 Gewichtsteile, meist auch 20 oder mehr Gewichtsteile auf eine oder mehrere Verbindungen der Formel (I). Es können jedoch auch erfindungsgemäße Dielektrika hergestellt werden, die ausschließlich aus zwei oder mehr Verbindungen der Formel (I) bestehen. Andererseits werden von der Erfindung auch solche flüssigkristallinen Dielektrika umfaßt, denen beispielsweise zu Dotierungszwecken nur weniger als 15 Gewichtsteile, zum Beispiel 0,1 bis 3 Gewichtsteile einer oder mehrerer Verbindungen der Formel (I) zugesetzt worden sind.

Die Herstellung der erfindungsgemäßen Dielektrika erfolgt in an sich üblicher Weise. In der Regel wird die gewünschte Menge einer oder mehrerer Verbindungen der Formel (I) in den gegebenenfalls anwesenden weiteren Komponenten gelöst, zweckmäßig bei erhöhter Temperatur. Wenn dabei eine Temperatur oberhalb des Klärpunkts des Basismaterials gewählt wird, kann die Vollständigkeit des Lösevorgangs besonders leicht beobachtet werden. Es ist jedoch auch möglich, Lösungen der Komponenten der Formeln (I) und ggf. (VIII) in einem geeigneten organischen Lösungsmittel, zum Beispiel Aceton, Chloroform oder Methanol, zu mischen und das Lösungsmittel nach gründlicher Durchmischung wieder zu entfernen, beispielsweise durch Destillation unter vermindertem Druck. Selbstverständlich muß bei dieser Verfahrensweise darauf geachtet werden, daß durch das Lösungsmittel keine Verunreinigungen oder unerwünschten Dotierungsstoffe eingeschleppt werden.

Durch geeignete Zusätze können die flüssigkristallinen Dielektrika nach der Erfindung so modifiziert werden, daß sie in allen bisher bekanntgewordenen Arten von Flüssigkristallanzeigeelementen verwendet werden können. Derartige Zusätze sind dem Fachmann bekannt und sind in der einschlägigen Literatur ausführlich beschrieben. Beispielsweise können Substanzen zur Veränderung der dielektrischen Anisotropie, der Viskosität, der Leitfähigkeit und/oder der Orientierung der nematischen Phasen zugesetzt werden. Derartige Substanzen sind zum Beispiel in den DE-OS 2 209 127, 2 240 864, 2 321 632, 2 338 281 und 2 450 088 beschrieben.

Die folgenden Beispiele sollen die Erfindung erläutern, ohne sie zu begrenzen. In den Beispielen bedeuten F. den Schmelzpunkt und K. den Klärpunkt einer flüssigkristallinen Substanz in Grad Celsius; Siedetemperaturen sind mit Kp. bezeichnet. Wenn nichts anderes angegeben ist, bedeuten Angaben von Teilen oder Prozent Gewichtsteile bzw. Gewichtsprozent.

Beispiel 1

Zu 1 l wasserfreiem Diäthyläther, der 23 g Natrium in Form eines Drahts enthält, wird eine Lösung von 265 g 4-(4-n-Pentylphenyl)-acetophenon in 300 ml Essigsäureäthylester gegeben und das Reaktionsgemisch 24 Stunden bei Raumtemperatur gerührt. Anschließend wird das ausgefallene Natriumsalz abfiltriert, mit Diäthyläther gewaschen und in ein Schüttelgefäß mit je 500 ml Diäthyläther und Wasser gegeben. Nach 1stündigem Ausschütteln wird die organische Phase abgetrennt, über Natriumsulfat getrocknet und eingedampft. Das zurückbleibende 1-[4-n-Pentylbiphenyl-(4')-yl]-butandion-(1,3) wird aus Äthanol umkristallisiert; F. 110°, K. 135°.

Analog werden hergestellt:

1-[4-(trans-4-Methylcyclohexyl)-phenyl]-butandion-(1,3)
1-[4-(trans-4-Äthylcyclohexyl)-phenyl]-butandion-(1,3)
1-[4-(trans-4-n-Propylcyclohexyl)-phenyl]-butandion-(1,3), F. 99,5°, K. 30°
1-[4-(trans-4-n-Butylcyclohexyl)-phenyl]-butandion-(1,3), F. 94°, K. 87°
1-[4-(trans-4-n-Pentylcyclohexyl)-phenyl]-butandion-(1,3), F. 81°, K. 97°
1-[4-(trans-4-n-Hexylcyclohexyl)-phenyl]-butandion-(1,3)
1-[4-(trans-4-n-Heptylcyclohexyl)-phenyl]-butandion-(1,3), F. 74°, K. 98°
1-[4-(trans-4-n-Octylcyclohexyl)-phenyl]-butandion-(1,3)
1-[4-(trans-4-n-Nonylcyclohexyl)-phenyl]-butandion-(1,3)
1-[4-(trans-4-n-Decylcyclohexyl)-phenyl]-butandion-(1,3)
1-[4-(trans-4-n-Undecylcyclohexyl)-phenyl]-butandion-(1,3)
1-[4-(trans-4-n-Dodecylcyclohexyl)-phenyl]-butandion-(1,3)
1-[4-(4-Methylcyclohexyl)-biphenyl-(4')-yl]-butandion-(1,3)
1-[4-(4-Äthylcyclohexyl)-biphenyl-(4')-yl]-butandion-(1,3)
1-[4-(4-n-Propylcyclohexyl)-biphenyl-(4')-yl]-butandion-(1,3)
1-[4-(4-n-Butylcyclohexyl)-biphenyl-(4')-yl]-butandion-(1,3)
1-[4-(4-n-Pentylcyclohexyl)-biphenyl-(4')-yl]-butandion-(1,3)
1-[4-(4-n-Hexylcyclohexyl)-biphenyl-(4')-yl]-butandion-(1,3)
1-[4-(4-n-Heptylcyclohexyl)-biphenyl-(4')-yl]-butandion-(1,3)
1-[4-(4-n-Octylcyclohexyl)-biphenyl-(4')-yl]-butandion-(1,3)
1-[4-(4-n-Nonylcyclohexyl)-biphenyl-(4')-yl]-butandion-(1,3)
1-[4-(4-n-Decylcyclohexyl)-biphenyl-(4')-yl]-butandion-(1,3)
1-[4-(4-n-Undecylcyclohexyl)-biphenyl-(4')-yl]-butandion-(1,3)
1-[4-(4-n-Dodecylcyclohexyl)-biphenyl-(4')-yl]-butandion-(1,3)
1-[4-(trans-4-Methylcyclohexyl)-phenyl]-pentandion-(1,3)
1-[4-(trans-4-Äthylcyclohexyl)-phenyl]-pentandion-(1,3)
1-[4-(trans-4-n-Propylcyclohexyl)-phenyl]-pentandion-(1,3), F. 78°, K. 84°
1-[4-(trans-4-n-Butylcyclohexyl)-phenyl]-pentandion-(1,3)
1-[4-(trans-4-n-Pentylcyclohexyl)-phenyl]-pentandion-(1,3), F. 59°, K. 91°
1-[4-(trans-4-n-Hexylcyclohexyl)-phenyl]-pentandion-(1,3)
1-[4-(trans-4-n-Heptylcyclohexyl)-phenyl]-pentandion-(1,3)
1-[4-(trans-4-n-Octylcyclohexyl)-phenyl]-pentandion-(1,3)
1-[4-(trans-4-n-Nonylcyclohexyl)-phenyl]-pentandion-(1,3)
1-[4-(trans-4-n-Decylcyclohexyl)-phenyl]-pentandion-(1,3)
1-[4-(trans-4-n-Undecylcyclohexyl)-phenyl]-pentandion-(1,3)
1-[4-(trans-4-n-Dodecylcyclohexyl)-phenyl]-pentandion-(1,3)
1-[4-(4-Methylcyclohexyl)-biphenyl-(4')-yl]-pentandion-(1,3)
1-[4-(4-Äthylcyclohexyl)-biphenyl-(4')-yl]-pentandion-(1,3)
1-[4-(4-n-Propylcyclohexyl)-biphenyl-(4')-yl]-pentandion-(1,3)
1-[4-(4-n-Butylcyclohexyl)-biphenyl-(4')-yl]-pentandion-(1,3)
1-[4-(4-n-Pentylcyclohexyl)-biphenyl-(4')-yl]-pentandion-(1,3)
1-[4-(4-n-Hexylcyclohexyl)-biphenyl-(4')-yl]-pentandion-(1,3)
1-[4-(4-n-Heptylcyclohexyl)-biphenyl-(4')-yl]-pentandion-(1,3)
1-[4-(4-n-Octylcyclohexyl)-biphenyl-(4')-yl]-pentandion-(1,3)
1-[4-(4-n-Nonylcyclohexyl)-biphenyl-(4')-yl]-pentandion-(1,3)
1-[4-(4-n-Decylcyclohexyl)-biphenyl-(4')-yl]-pentandion-(1,3)
1-[4-(4-n-Undecylcyclohexyl)-biphenyl-(4')-yl]-pentandion-(1,3)
1-[4-(4-n-Dodecylcyclohexyl)-biphenyl-(4')-yl]-pentandion-(1,3)
1-[4-(trans-4-Methylcyclohexyl)-phenyl]-hexandion-(1,3)
1-[4-(trans-4-Äthylcyclohexyl)-phenyl]-hexandion-(1,3)
1-[4-(trans-4-n-Propylcyclohexyl)-phenyl]-hexandion-(1,3), F. 99°, K. 100°
1-[4-(trans-4-n-Butylcyclohexyl)-phenyl]-hexandion-(1,3)
1-[4-(trans-4-n-Pentylcyclohexyl)-phenyl]-hexandion-(1,3)
1-[4-(trans-4-n-Hexylcyclohexyl)-phenyl]-hexandion-(1,3)

1-[4-(trans-4-n-Heptylcyclohexyl)-phenyl]-hexandion-(1,3)
1-[4-(trans-4-n-Octylcyclohexyl)-phenyl]-hexandion-(1,3)
1-[4-(trans-4-n-Nonylcyclohexyl)-phenyl]-hexandion-(1,3)
1-[4-(trans-4-n-Decylcyclohexyl)-phenyl]-hexandion-(1,3)
1-[4-(trans-4-n-Undecylcyclohexyl)-phenyl]-hexandion-(1,3)
1-[4-(trans-4-n-Dodecylcyclohexyl)-phenyl]-hexandion-(1,3)
1-[4-(4-Methylcyclohexyl)-biphenyl-(4')-yl]-hexandion-(1,3)
1-[4-(4-Äthylcyclohexyl)-biphenyl-(4')-yl]-hexandion-(1,3)
1-[4-(4-n-Propylcyclohexyl)-biphenyl-(4')-yl]-hexandion-(1,3)
1-[4-(4-n-Butylcyclohexyl)-biphenyl-(4')-yl]-hexandion-(1,3)
1-[4-(4-n-Pentylcyclohexyl)-biphenyl-(4')-yl]-hexandion-(1,3)
1-[4-(4-n-Hexylcyclohexyl)-biphenyl-(4')-yl]-hexandion-(1,3)
1-[4-(4-n-Heptylcyclohexyl)-biphenyl-(4')-yl]-hexandion-(1,3)
1-[4-(4-n-Octylcyclohexyl)-biphenyl-(4')-yl]-hexandion-(1,3)
1-[4-(4-n-Nonylcyclohexyl)-biphenyl-(4')-yl]-hexandion-(1,3)
1-[4-(4-n-Decylcyclohexyl)-biphenyl-(4')-yl]-hexandion-(1,3)
1-[4-(4-n-Undecylcyclohexyl)-biphenyl-(4')-yl]-hexandion-(1,3)
1-[4-(4-n-Dodecylcyclohexyl)-biphenyl-(4')-yl]-hexandion-(1,3)
1-[4-(trans-4-Methylcyclohexyl)-phenyl]-heptandion-(1,3)
1-[4-(trans-4-Äthylcyclohexyl)-phenyl]-heptandion-(1,3)
1-[4-(trans-4-n-Propylcyclohexyl)-phenyl]-heptandion-(1,3), F. 90°, K. 75°
1-[4-(trans-4-n-Butylcyclohexyl)-phenyl]-heptandion-(1,3)
1-[4-(trans-4-n-Pentylcyclohexyl)-phenyl]-heptandion-(1,3)
1-[4-(trans-4-n-Hexylcyclohexyl)-phenyl]-heptandion-(1,3)
1-[4-(trans-4-n-Heptylcyclohexyl)-phenyl]-heptandion-(1,3)
1-[4-(trans-4-n-Octylcyclohexyl)-phenyl]-heptandion-(1,3)
1-[4-(trans-4-n-Nonylcyclohexyl)-phenyl]-heptandion-(1,3)
1-[4-(trans-4-n-Decylcyclohexyl)-phenyl]-heptandion-(1,3)
1-[4-(trans-4-n-Undecylcyclohexyl)-phenyl]-heptandion-(1,3)
1-[4-(trans-4-n-Dodecylcyclohexyl)-phenyl]-heptandion-(1,3)
1-[4-(4-Methylcyclohexyl)-biphenyl-(4')-yl]-heptandion-(1,3)
1-[4-(4-Äthylcyclohexyl)-biphenyl-(4')-yl]-heptandion-(1,3)
1-[4-(4-n-Propylcyclohexyl)-biphenyl-(4')-yl]-heptandion-(1,3)
1-[4-(4-n-Butylcyclohexyl)-biphenyl-(4')-yl]-heptandion-(1,3)
1-[4-(4-n-Pentylcyclohexyl)-biphenyl-(4')-yl]-heptandion-(1,3)
1-[4-(4-n-Hexylcyclohexyl)-biphenyl-(4')-yl]-heptandion-(1,3)
1-[4-(4-n-Heptylcyclohexyl)-biphenyl-(4')-yl]-heptandion-(1,3)
1-[4-(4-n-Octylcyclohexyl)-biphenyl-(4')-yl]-heptandion-(1,3)
1-[4-(4-n-Nonylcyclohexyl)-biphenyl-(4')-yl]-heptandion-(1,3)
1-[4-(4-n-Decylcyclohexyl)-biphenyl-(4')-yl]-heptandion-(1,3)
1-[4-(4-n-Undecylcyclohexyl)-biphenyl-(4')-yl]-heptandion-(1,3)
1-[4-(4-n-Dodecylcyclohexyl)-biphenyl-(4')-yl]-heptandion-(1,3)
1-[4-(trans-4-Methylcyclohexyl)-phenyl]-4,4,4-trifluorbutandion-(1,3)
1-[4-(trans-4-Äthylcyclohexyl)-phenyl]-4,4,4-trifluorbutandion-(1,3)
1-[4-(trans-4-n-Propylcyclohexyl)-phenyl]-4,4,4-trifluorbutandion-(1,3)
1-[4-(trans-4-n-Butylcyclohexyl)-phenyl]-4,4,4-trifluorbutandion-(1,3)
1-[4-(trans-4-n-Pentylcyclohexyl)-phenyl]-4,4,4-trifluorbutandion-(1,3), F. 65°, K. 40°
1-[4-(trans-4-n-Hexylcyclohexyl)-phenyl]-4,4,4-trifluorbutandion-(1,3)
1-[4-(trans-4-n-Heptylcyclohexyl)-phenyl]-4,4,4-trifluorbutandion-(1,3)
1-[4-(trans-4-n-Octylcyclohexyl)-phenyl]-4,4,4-trifluorbutandion-(1,3)
1-[4-(trans-4-n-Nonylcyclohexyl)-phenyl]-4,4,4-trifluorbutandion-(1,3)
1-[4-(trans-4-n-Decylcyclohexyl)-phenyl]-4,4,4-trifluorbutandion-(1,3)
1-[4-(trans-4-n-Undecylcyclohexyl)-phenyl]-4,4,4-trifluorbutandion-(1,3)
1-[4-(trans-4-n-Dodecylcyclohexyl)-phenyl]-4,4,4-trifluorbutandion-(1,3)
1-[4-(4-Methylcyclohexyl)-biphenyl-(4')-yl]-4,4,4-trifluorbutandion-(1,3)
1-[4-(4-Äthylcyclohexyl)-biphenyl-(4')-yl]-4,4,4-trifluorbutandion-(1,3)
1-[4-(4-n-Propylcyclohexyl)-biphenyl-(4')-yl]-4,4,4-trifluorbutandion-(1,3)
1-[4-(4-n-Butylcyclohexyl)-biphenyl-(4')-yl]-4,4,4-trifluorbutandion-(1,3)
1-[4-(4-n-Pentylcyclohexyl)-biphenyl-(4')-yl]-4,4,4-trifluorbutandion-(1,3)
1-[4-(4-n-Hexylcyclohexyl)-biphenyl-(4')-yl]-4,4,4-trifluorbutandion-(1,3)
1-[4-(4-n-Heptylcyclohexyl)-biphenyl-(4')-yl]-4,4,4-trifluorbutandion-(1,3)
1-[4-(4-n-Octylcyclohexyl)-biphenyl-(4')-yl]-4,4,4-trifluorbutandion-(1,3)
1-[4-(4-n-Nonylcyclohexyl)-biphenyl-(4')-yl]-4,4,4-trifluorbutandion-(1,3)
1-[4-(4-n-Decylcyclohexyl)-biphenyl-(4')-yl]-4,4,4-trifluorbutandion-(1,3)
1-[4-(4-n-Undecylcyclohexyl)-biphenyl-(4')-yl]-4,4,4-trifluorbutandion-(1,3)

1-[4-(4-n-Dodecylcyclohexyl)-biphenyl-(4')-yl]-4,4,4-trifluorbutandion-(1,3)
1-[4-Methylbiphenyl-(4')-yl]-butandion-(1,3)
1-[4-Äthylbiphenyl-(4')-yl]-butandion-(1,3)
1-[4-n-Propylbiphenyl-(4')-yl]-butandion-(1,3)
1-[4-n-Butylbiphenyl-(4')-yl]-butandion-(1,3)
1-[4-n-Hexylbiphenyl-(4')-yl]-butandion-(1,3)
1-[4-n-Heptylbiphenyl-(4')-yl]-butandion-(1,3)
1-[4-n-Octylbiphenyl-(4')-yl]-butandion-(1,3)
1-[4-n-Nonylbiphenyl-(4')-yl]-butandion-(1,3)
1-[4-n-Decylbiphenyl-(4')-yl]-butandion-(1,3)
1-[4-n-Undecylbiphenyl-(4')-yl]-butandion-(1,3)
1-[4-n-Dodecylbiphenyl-(4')-yl]-butandion-(1,3)
1-[4-Trifluormethylbiphenyl-(4')-yl]-butandion-(1,3)
1-[4-Methoxybiphenyl-(4')-yl]-butandion-(1,3)
1-[4-Äthoxybiphenyl-(4')-yl]-butandion-(1,3)
1-[4-n-Propyloxybiphenyl-(4')-yl]-butandion-(1,3)
1-[4-n-Butyloxybiphenyl-(4')-yl]-butandion-(1,3)
1-[4-n-Pentyloxybiphenyl-(4')-yl]-butandion-(1,3)
1-[4-n-Hexyloxybiphenyl-(4')-yl]-butandion-(1,3)
1-[4-n-Heptyloxybiphenyl-(4')-yl]-butandion-(1,3)
1-[4-n-Octyloxybiphenyl-(4')-yl]-butandion-(1,3)
1-[4-n-Nonyloxybiphenyl-(4')-yl]-butandion-(1,3)
1-[4-n-Decyloxybiphenyl-(4')-yl]-butandion-(1,3)
1-[4-n-Undecyloxybiphenyl-(4')-yl]-butandion-(1,3)
1-[4-n-Dodecyloxybiphenyl-(4')-yl]-butandion-(1,3)
1-[4-Methylbiphenyl-(4')-yl]-pentandion-(1,3)
1-[4-Äthylbiphenyl-(4')-yl]-pentandion-(1,3)
1-[4-n-Propylbiphenyl-(4')-yl]-pentandion-(1,3)
1-[4-n-Butylbiphenyl-(4')-yl]-pentandion-(1,3)
1-[4-n-Pentylbiphenyl-(4')-yl]-pentandion-(1,3)
1-[4-n-Hexylbiphenyl-(4')-yl]-pentandion-(1,3)
1-[4-n-Heptylbiphenyl-(4')-yl]-pentandion-(1,3)
1-[4-n-Octylbiphenyl-(4')-yl]-pentandion-(1,3)
1-[4-n-Nonylbiphenyl-(4')-yl]-pentandion-(1,3)
1-[4-n-Decylbiphenyl-(4')-yl]-pentandion-(1,3)
1-[4-n-Undecylbiphenyl-(4')-yl]-pentandion-(1,3)
1-[4-n-Dodecylbiphenyl-(4')-yl]-pentandion-(1,3)
1-[4-Trifluormethylbiphenyl-(4')-yl]-pentandion-(1,3)
1-[4-Methoxybiphenyl-(4')-yl]-pentandion-(1,3)
1-[4-Äthoxybiphenyl-(4')-yl]-pentandion-(1,3)
1-[4-n-Propyloxybiphenyl-(4')-yl]-pentandion-(1,3)
1-[4-n-Butyloxybiphenyl-(4')-yl]-pentandion-(1,3)
1-[4-n-Pentyloxybiphenyl-(4')-yl]-pentandion-(1,3)
1-[4-n-Hexyloxybiphenyl-(4')-yl]-pentandion-(1,3)
1-[4-n-Heptyloxybiphenyl-(4')-yl]-pentandion-(1,3)
1-[4-n-Octyloxybiphenyl-(4')-yl]-pentandion-(1,3)
1-[4-n-Nonyloxybiphenyl-(4')-yl]-pentandion-(1,3)
1-[4-n-Decyloxybiphenyl-(4')-yl]-pentandion-(1,3)
1-[4-n-Undecyloxybiphenyl-(4')-yl]-pentandion-(1,3)
1-[4-n-Dodecyloxybiphenyl-(4')-yl]-pentandion-(1,3)
1-[4-Methylbiphenyl-(4')-yl]-hexandion-(1,3)
1-[4-Äthylbiphenyl-(4')-yl]-hexandion-(1,3)
1-[4-n-Propylbiphenyl-(4')-yl]-hexandion-(1,3)
1-[4-n-Butylbiphenyl-(4')-yl]-hexandion-(1,3)
1-[4-n-Pentylbiphenyl-(4')-yl]-hexandion-(1,3)
1-[4-n-Hexylbiphenyl-(4')-yl]-hexandion-(1,3)
1-[4-n-Heptylbiphenyl-(4')-yl]-hexandion-(1,3)
1-[4-n-Octylbiphenyl-(4')-yl]-hexandion-(1,3)
1-[4-n-Nonylbiphenyl-(4')-yl]-hexandion-(1,3)
1-[4-n-Decylbiphenyl-(4')-yl]-hexandion-(1,3)
1-[4-n-Undecylbiphenyl-(4')-yl]-hexandion-(1,3)
1-[4-n-Dodecylbiphenyl-(4')-yl]-hexandion-(1,3)
1-[4-Trifluormethylbiphenyl-(4')-yl]-hexandion-(1,3)
1-[4-Methoxybiphenyl-(4')-yl]-hexandion-(1,3)
1-[4-Äthoxybiphenyl-(4')-yl]-hexandion-(1,3)

1-[4-n-Propyloxybiphenyl-(4')-yl]-hexandion-(1,3)
1-[4-n-Butyloxybiphenyl-(4')-yl]-hexandion-(1,3)
1-[4-n-Pentyloxybiphenyl-(4')-yl]-hexandion-(1,3)
1-[4-n-Hexyloxybiphenyl-(4')-yl]-hexandion-(1,3)
1-[4-n-Heptyloxybiphenyl-(4')-yl]-hexandion-(1,3)
1-[4-n-Octyloxybiphenvl-(4')-yl]-hexandion-(1,3)
1-[4-n-Nonyloxybiphenyl-(4')-yl]-hexandion-(1,3)
1-[4-n-Decyloxybiphenyl-(4')-yl]-hexandion-(1,3)
1-[4-n-Undecyloxybiphenyl-(4')-yl]-hexandion-(1,3)
1-[4-n-Dodecyloxybiphenyl-(4')-yl]-hexandion-(1,3)
1-[4-Methylbiphenyl-(4')-yl]-heptandion-(1,3)
1-[4-Äthylbiphenyl-(4')-yl]-heptandion-(1,3)
1-[4-n-Propylbiphenyl-(4')-yl]-heptandion-(1,3)
1-[4-n-Butylbiphenyl-(4')-yl]-heptandion-(1,3)
1-[4-n-Pentylbiphenyl-(4')-yl]-heptandion-(1,3)
1-[4-n-Hexylbiphenyl-(4')-yl]-heptandion-(1,3)
1-[4-n-Heptylbiphenyl-(4')-yl]-heptandion-(1,3)
1-[4-n-Octylbiphenyl-(4')-yl]-heptandion-(1,3)
1-[4-n-Nonylbiphenyl-(4')-yl]-heptandion-(1,3)
1-[4-n-Decylbiphenyl-(4')-yl]-heptandion-(1,3)
1-[4-n-Undecylbiphenyl-(4')-yl]-heptandion-(1,3)
1-[4-n-Dodecylbiphenyl-(4')-yl]-heptandion-(1,3)
1-[4-Trifluormethylbiphenyl-(4')-yl]-heptandion-(1,3)
1-[4-Methoxybiphenyl-(4')-yl]-heptandion-(1,3)
1-[4-Äthoxybiphenyl-(4')-yl]-heptandion-(1,3)
1-[4-n-Propyloxybiphenyl-(4')-yl]-heptandion-(1,3)
1-[4-n-Butyloxybiphenyl-(4')-yl]-heptandion-(1,3)
1-[4-n-Pentyloxybiphenyl-(4')-yl]-heptandion-(1,3)
1-[4-n-Hexyloxybiphenyl-(4')-yl]-heptandion-(1,3)
1-[4-n-Heptyloxybiphenyl-(4')-yl]-heptandion-(1,3)
1-[4-n-Octyloxybiphenyl-(4')-yl]-heptandion-(1,3)
1-[4-n-Nonyloxybiphenyl-(4')-yl]-heptandion-(1,3)
1-[4-n-Decyloxybiphenyl-(4')-yl]-heptandion-(1,3)
1-[4-n-Undecyloxybiphenyl-(4')-yl]-heptandion-(1,3)
1-[4-n-Dodecyloxybiphenyl-(4')-yl]-heptandion-(1,3)
1-[4-Methylbiphenyl-(4')-yl]-4,4,4-trifluorbutandion-(1,3)
1-[4-Äthylbiphenyl-(4')-yl]-4,4,4-trifluorbutandion-(1,3)
1-[4-n-Propylbiphenyl-(4')-yl]-4,4,4-trifluorbutandion-(1,3)
1-[4-n-Butylbiphenyl-(4')-yl]-4,4,4-trifluorbutandion-(1,3)
1-[4-n-Pentylbiphenyl-(4')-yl]-4,4,4-trifluorbutandion-(1,3)
1-[4-n-Hexylbiphenyl-(4')-yl]-4,4,4-trifluorbutandion-(1,3)
1-[4-n-Heptylbiphenyl-(4')-yl]-4,4,4-trifluorbutandion-(1,3)
1-[4-n-Octylbiphenyl-(4')-yl]-4,4,4-trifluorbutandion-(1,3)
1-[4-n-Nonylbiphenyl-(4')-yl]-4,4,4-trifluorbutandion-(1,3)
1-[4-n-Decylbiphenyl-(4')-yl]-4,4,4-trifluorbutandion-(1,3)
1-[4-n-Undecylbiphenyl-(4')-yl]-4,4,4-trifluorbutandion-(1,3)
1-[4-n-Dodecylbiphenyl-(4')-yl]-4,4,4-trifluorbutandion-(1,3)
1-[4-Trifluormethylbiphenyl-(4')-yl]-4,4,4-trifluorbutandion-(1,3)
1-[4-Methoxybiphenyl-(4')-yl]-4,4,4-trifluorbutandion-(1,3)
1-[4-Äthoxybiphenyl-(4')-yl]-4,4,4-trifluorbutandion-(1,3)
1-[4-n-Propyloxybiphenyl-(4')-yl]-4,4,4-trifluorbutandion-(1,3)
1-[4-n-Butyloxybiphenyl-(4')-yl]-4,4,4-trifluorbutandion-(1,3)
1-[4-n-Pentyloxybiphenyl-(4')-yl]-4,4,4-trifluorbutandion-(1,3)
1-[4-n-Hexyloxybiphenyl-(4')-yl]-4,4,4-trifluorbutandion-(1,3)
1-[4-n-Heptyloxybiphenyl-(4')-yl]-4,4,4-trifluorbutandion-(1,3)
1-[4-n-Octyloxybiphenyl-(4')-yl]-4,4,4-trifluorbutandion-(1,3)
1-[4-n-Nonyloxybiphenyl-(4')-yl]-4,4,4-trifluorbutandion-(1,3)
1-[4-n-Decyloxybiphenyl-(4')-yl]-4,4,4-trifluorbutandion-(1,3)
1-[4-n-Undecyloxybiphenyl-(4')-yl]-4,4,4-trifluorbutandion-(1,3)
1-[4-n-Dodecyloxybiphenyl-(4')-yl]-4,4,4-trifluorbutandion-(1,3)

Die folgenden Beispiele betreffen die erfindungsgemäße Verwendung der Diketone (I) als Komponenten flüssigkristalliner Dielektrika:

Beispiel 2

Eine Mischung aus

46,7 Gewichtsteilen Anissäure-4-n-pentylphenylester,

23,3 Gewichtsteilen 4-n-Hexyloxybenzoesäure-4'-n-pentylphenylester,

15,0 Gewichtsteilen 1-[4-(trans-4-n-Pentylcyclohexyl)-phenyl]-pentandion-(1,3),

7,5 Gewichtsteilen 1-[4-(trans-4-n-Pentylcyclohexyl)-phenyl]-butandion-(1,3), und

7,5 Gewichtsteilen 1-[4-(trans-4-n-Butylcyclohexyl)-phenyl]-butandion-(1,3)

hat einen Schmelzpunkt von 5° und einen Klärpunkt von 64°. In dieser Mischung können ohne Schwierigkeiten 5 Gewichtsteile des dichroitischen Farbstoffs 1-Hydroxy-4-N-(p-n-nonyloxyphenyl)-amino-anthrachinon bei 25° gelöst werden.

Beispiel 3

Eine Mischung aus

58,0 Gewichtsteilen 4-n-Butyl-4'-methoxyazoxybenzol,

29,0 Gewichtsteilen 4-Äthyl-4'-methoxyazoxybenzol,

8,0 Gewichtsteilen 4-n-Heptylbiphenyl-(1)-carbonsäure-2'-cyano-4'-n-heptylphenylester und

5,0 Gewichtsteilen 1-[4-(trans-4-n-Pentylcyclohexyl)-phenyl]-butandion-(1,3)

hat einen Schmelzpunkt von −4° und einen Klärpunkt von 78°. In der Mischung ist Äthyl-dodecyl-dimethyl-ammonium-4-(4-n-hexyloxybenzol)-benzoat gut löslich; das so erhaltene Dielektrikum ist für Flüssigkristall Anzeigeelemente auf der Basis des dynamischen Streueffekts hervorragend geeignet. Ebenso ist der dichroitische Farbstoff 1-Hydroxy-4-N-(p-n-nonyloxyphenyl)-amino-anthrachinon in dieser Mischung in Mengen bis zu 5% gut löslich; diese farbstoffhaltigen Dielektrika sind besonders gut zur Herstellung von farbigen Anzeigeelementen geeignet, die auf der Basis der Deformation aufgerichteter Phasen (DAP-Effekt) arbeiten.

Beispiel 4

Eine Mischung aus

26,1 Gewichtsteilen 4-(4-n-Propylcyclohexyl)-benzonitril,

36,9 Gewichtsteilen 4-(4-n-Pentylcyclohexyl)-benzonitril,

27,0 Gewichtsteilen 4-(4-n-Heptylcyclohexyl)-benzonitril,

5,0 Gewichtsteilen 1-[4-(trans-4-n-Propylcyclohexyl)-phenyl]-4,4,4-trifluorbutandion-(1,3) und

5,0 Gewichtsteilen 1-[4-(trans-4-n-Pentylcyclohexyl)-phenyl]-4,4,4-trifluorbutandion-(1,3)

hat einen Schmelzpunkt von 0° und einen Klärpunkt von 50°. In dieser Mischung sind dichroitische Farbstoffe wie zum Beispiel 1-Hydroxy-4-N-(p-n-nonyloxyphenyl)-aminoanthrachinon gut löslich; die dadurch erhaltenen Dielektrika ermöglichen nach üblicher Dotierung mit einer chiralen Substanz die Herstellung von Flüssigkristall-Farbschaltern mit gutem Kontrast.

Beispiel 5

Eine Mischung aus

14 Gewichtsteilen 1-[4-(trans-4-n-Butylcyclohexyl)-phenyl]-butandion-(1,3),

14 Gewichtsteilen 1-[4-(trans-4-n-Pentylcyclohexyl)-phenyl]-butandion-(1,3),

14 Gewichtsteilen 1-[4-(trans-4-n-Heptylcyclohexyl)-phenyl]-butandion-(1,3),

29 Gewichtsteilen 1-[4-(trans-4-n-Pentylcyclohexyl)-phenyl]-pentandion-(1,3) und

29 Gewichtsteilen 1-(4-Heptylphenyl)-butandion-(1,3)

hat einen Schmelzpunkt von 20° und einen Klärpunkt von 49°. Die Mischung besitzt eine negative dielektrische Anisotropie und ein gutes Lösungsvermögen für dichroitische Farbstoffe oder Acetylacetonat-Komplexe mehrwertiger Metallionen; derartige Lösungen besitzen eine elektrische

Leitfähigkeit, die einerseits von den Metall-Kationen und andererseits von den in der flüssig-kristallinen Matrix enthaltenen Keto-Enolat-Anionen getragen wird.

**Patentansprüche für die Vertragsstaaten: BE, CH, DE, FR, GB, IT, NL, SE**

1. Diketone der Formel (I)

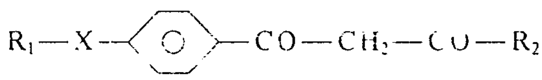 (I)

worin
$R_1$ Alkyl, Perfluoralkyl oder Alkoxy mit 1 bis 18 C-Atomen,
$R_2$ Alkyl oder Perfluoralkyl mit 1 bis 12 C-Atomen und
X 1,4-trans-Cyclohexylen, 1,4-Phenylen oder 4,4'-Cyclohexylphenyl
bedeutet.

2. Diketone nach Anspruch 1 der Formel (Ia)

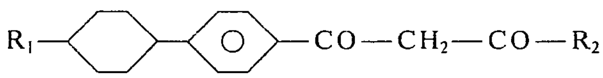 (Ia)

worin
$R_1$ Alkyl mit 1 bis 18 C-Atomen und
$R_2$ Alkyl oder Perfluoralkyl mit 1 bis 12 C-Atomen
bedeutet.

3. Diketone nach Anspruch 1 der Formel (Ib)

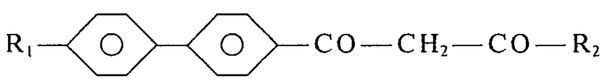 (Ib)

worin
$R_1$ Alkyl, Perfluoralkyl oder Alkoxy mit 1 bis 18 C-Atomen und
$R_2$ Alkyl oder Perfluoralkyl mit 1 bis 12 C-Atomen
bedeutet.

4. Diketone nach Anspruch 1 der Formel (Ic)

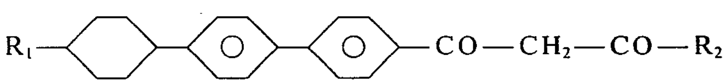 (Ic)

worin
$R_1$ Alkyl mit 1 bis 18 C-Atomen und
$R_2$ Alkyl oder Perfluoralkyl mit 1 bis 12 C-Atomen
bedeutet.

5. Verfahren zur Herstellung der Diketone der Formel (I)

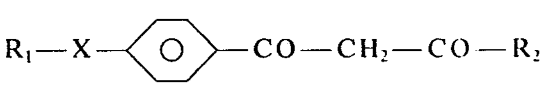 (I)

worin
$R_1$ Alkyl, Perfluoralkyl oder Alkoxy mit 1 bis 18 C-Atomen,
$R_2$ Alkyl oder Perfluoralkyl mit 1 bis 12 C-Atomen und
X 1,4-trans-Cyclohexylen, 1,4-Phenylen oder 4,4'-Cyclohexylphenyl
bedeutet,
dadurch gekennzeichnet, daß ein Acetophenon der Formel (II)

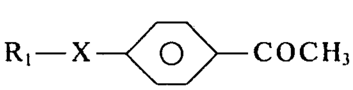 (II)

worin
$R_1$ die vorstehend angegebene Bedeutung besitzt,

in Gegenwart eines basischen Kondensationsmittels mit einem Carbonsäureester der Formel (III)

$$R_2—COO—Z \qquad (III)$$

worin
Z eine niedere Alkylgruppe ist und
$R_2$ die vorstehend angegebene Bedeutung besitzt,
umsetzt und anschließend das entstandene Salz der Enol-Form des Diketons (I) zu diesem hydrolysiert.

6. Verwendung eines Diketons der Formel (I)

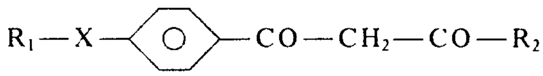
(I)

worin
$R_1$ Alkyl, Perfluoralkyl oder Alkoxy mit 1 bis 18 C-Atomen,
$R_2$ Alkyl oder Perfluoralkyl mit 1 bis 12 C-Atomen und
X 1,4-trans-Cyclohexylen, 1,4-Phenylen oder 4,4'-Cyclohexylphenyl
bedeutet,
als Komponente eines flüssigkristallinen Dielektrikums.

7. Flüssigkristallines Dielektrikum, dadurch gekennzeichnet, daß es mindestens ein Diketon der Formel (I) enthält,

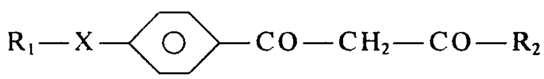
(I)

worin
$R_1$ Alkyl, Perfluoralkyl oder Alkoxy mit 1 bis 18 C-Atomen,
$R_2$ Alkyl oder Perfluoralkyl mit 1 bis 12 C-Atomen und
X 1,4-trans-Cyclohexylen, 1,4-Phenylen oder 4,4'-Cyclohexylphenyl
bedeutet.

8. Elektrooptisches Anzeigeelement auf der Basis einer Flüssigkristallzelle, dadurch gekennzeichnet, daß es ein flüssigkristallines Dielektrikum nach Anspruch 7 enthält.

**Patentansprüche für den Vertragsstaat: AT**

1. Verfahren zur Herstellung der Diketone der Formel (I)

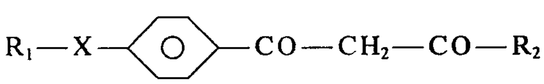
(I)

worin
$R_1$ Alkyl, Perfluoralkyl oder Alkoxy mit 1 bis 18 C-Atomen,
$R_2$ Alkyl oder Perfluoralkyl mit 1 bis 12 C-Atomen und
X 1,4-trans-Cyclohexylen, 1,4-Phenylen oder 4,4'-Cyclohexylphenyl
bedeutet,
dadurch gekennzeichnet, daß ein Acetophenon der Formel (II)

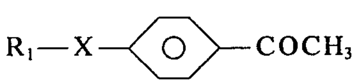
(II)

worin
$R_1$ die vorstehend angegebene Bedeutung besitzt,
in Gegenwart eines basischen Kondensationsmittels mit einem Carbonsäureester der Formel (III)

$$R_2—COO—Z \qquad (III)$$

worin
Z eine niedere Alkylgruppe ist und
$R_2$ die vorstehend angegebene Bedeutung besitzt,

umsetzt und anschließend das entstandene Salz der Enol-Form des Diketons (I) zu diesem hydrolysiert.

2. Verwendung eines Diketons der Formel (I)

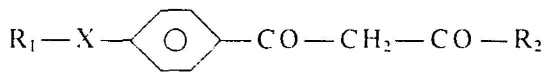 (I)

worin

$R_1$ Alkyl, Perfluoralkyl oder Alkoxy mit 1 bis 18 C-Atomen,

$R_2$ Alkyl oder Perfluoralkyl mit 1 bis 12 C-Atomen und

X 1,4-trans-Cyclohexylen, 1,4-Phenylen oder 4,4'-Cyclohexylphenyl

bedeutet,

als Komponente eines flüssigkristallinen Dielektrikums.

3. Flüssigkristallines Dielektrikum, dadurch gekennzeichnet, daß es mindestens ein Diketon der Formel (I) enthält,

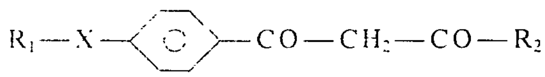 (I)

worin

$R_1$ Alkyl, Perfluoralkyl oder Alkoxy mit 1 bis 18 C-Atomen,

$R_2$ Alkyl oder Perfluoralkyl mit 1 bis 12 C-Atomen und

X 1,4-trans-Cyclohexylen, 1,4-Phenylen oder 4,4'-Cyclohexylphenyl

bedeutet.

4. Elektrooptisches Anzeigeelement auf der Basis einer Flüssigkristallzelle, dadurch gekennzeichnet, daß es ein flüssigkristallines Dielektrikum nach Anspruch 3 enthält.

**Claims for the Contracting States: BE, CH, DE, FR, GB, IT, NL, SE**

1. Diketones of the formula (I)

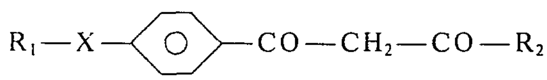 (I)

in which $R_1$ is alkyl, perfluoroalkyl or alkoxy with 1−18 C-atoms, $R_2$ is alkyl or perfluoroalkyl with 1−12 C-atoms and X is 1,4-trans-cyclohexylene, 1,4-phenylene, or 4,4'-cyclohexylphenyl.

2. Diketones according to Claim 1, of the formula (Ia)

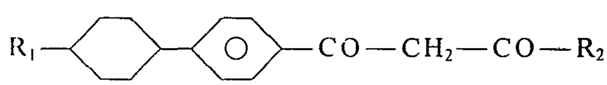 (Ia)

in which $R_1$ is alkyl with 1−18 C-atoms and $R_2$ is alkyl or perfluoroalkyl with 1−12 C-atoms.

3. Diketones according to Claim 1, of the formula (Ib)

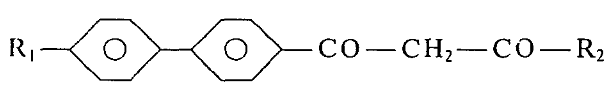 (Ib)

in which $R_1$ is alkyl, perfluoralkyl or alkoxy with 1−18 C-atoms and $R_2$ is alkyl or perfluoroalkyl with 1−12 C-atoms.

4. Diketones according to Claim 1, of the formula (Ic)

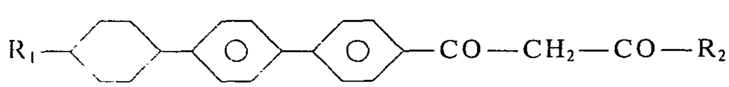 (Ic)

in which $R_1$ is alkyl with 1−18 C-atoms and $R_2$ is alkyl or perfluoroalkyl with 1−12 C-atoms.

5. Process for the preparation of the diketones of the formula (I)

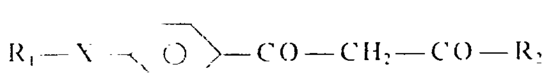 (I)

in which $R_1$ is alkyl, perfluoroalkyl or alkoxy with 1−18 C-atoms, $R_2$ is alkyl or perfluoroalkyl with 1−12 C-atoms and X is 1,4-trans-cyclohexylene, 1,4-phenylene, or 4,4′-cyclohexylphenyl, characterised in that an acetophenone of the formula (II)

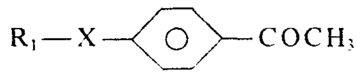 (II)

in which $R_1$ is as defined above, is reacted in the presence of a basic condensing agent with a carboxylic acid ester of the formula (III)

$$R_2—COO—Z \qquad (III)$$

in which Z is a lower akyl group and $R_2$ is as defined above, and the resulting salt of the enol form of the diketone (I) is then hydrolysed to this diketone.

6. Use of a diketone of the formula (I)

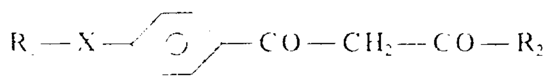 (I)

in which $R_1$ is alkyl, perfluoroalkyl or alkoxy with 1−18 C-atoms, $R_2$ is alkyl or perfluoroalkyl with 1−12 C-atoms and X is 1,4-trans-cyclohexylene, 1,4-phenylene, or 4,4′-cyclohexylphenyl, as a component of a liquid crystal dielectric.

7. Liquid crystal dielectric, characterised in that it contains at least one diketone of the formula (I),

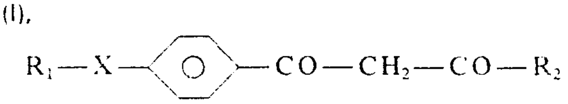 (I)

in which $R_1$ is alkyl, perfluoroalkyl or alkoxy with 1−18 C-atoms, $R_2$ is alkyl or perfluoroalkyl with 1−12 C-atoms and X is 1,4-trans-cyclohexylene, 1,4-phenylene, or 4,4′-cyclohexylphenyl.

8. Electro-optical display element based on a liquid crystal cell, characterised in that it contains a liquid crystal dielectric according to Claim 7.

**Claims for the Contracting State: AT**

1. Process for the preparation of the diketones of the formula (I)

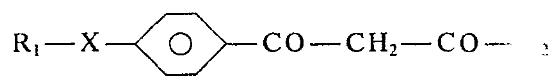 (I)

in which $R_1$ is alkyl, perfluoroalkyl or alkoxy with 1−18 C-atoms, $R_2$ is alkyl or perfluoroalkyl with 1−12 C-atoms and X is 1,4-trans-cyclohexylene, 1,4-phenylene, or 4,4′-cyclohexylphenyl, characterised in that an acetophenone of the formula (II)

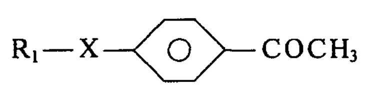 (II)

in which $R_1$ is as defined above, is reacted in the presence of a basic condensing agent with a carboxylic acid ester of the formula (III)

$$R_2—COO—Z \qquad (III)$$

in which Z is a lower alkyl group and $R_2$ is as defined above, and the resulting salt of the enol form of the diketone (I) is then hydrolysed to this diketone.

2. Use of a diketone of the formula (I)

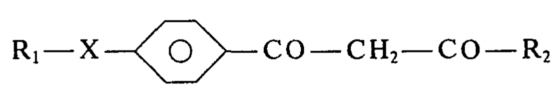 (I)

in which $R_1$ is alkyl, perfluoroalkyl or alkoxy with 1−18 C-atoms, $R_2$ is alkyl or perfluoroalkyl with 1−12 C-atoms and X is 1,4-trans-cyclohexylene, 1,4-phenylene, or 4,4′-cyclohexylphenyl, as a component of a liquid crystal dielectric.

3. Liquid crystal dielectric, characterised in that it contains at least one diketone of the formula (I),

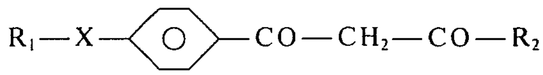

(I)

in which $R_1$ is alkyl, perfluoroalkyl or alkoxy with 1—18 C-atoms, $R_2$ is alkyl or perfluoroalkyl with 1—12 C-atoms and X is 1,4-trans-cyclohexylene, 1,4-phenylene, or 4,4'-cyclohexylphenyl.

4. Electro-optical display element based on a liquid crystal cell, characterised in that it contains a liquid crystal dielectric according to Claim 3.

**Revendications pour les Etats contractants: BE, CH, DE, FR, GB, IT, NL, SE**

1. Dicétones de formule (I)

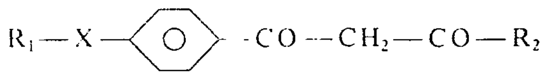

(I)

où

$R_1$ représente un alcoyle, un perfluoroalcoyle ou un alcoxy en $C_1$ à $C_{18}$,

$R_2$ représente un alcoyle ou un perfluoroalcoyle en $C_1$ à $C_{12}$ et

X représente un 1,4-trans-cyclohexylène, un 1,4-phénylène ou un 4,4'-cyclohexylphényle.

2. Dicétones selon la revendication 1 de formule (Ia)

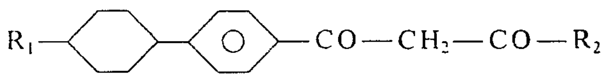

(Ia)

où

$R_1$ représente un alcoyle en $C_1$ à $C_{18}$ et

$R_2$ represente un alcoyle ou un perfluoroalcoyle en $C_1$ à $C_{12}$.

3. Dicétones selon la revendication 1 de formule (Ib)

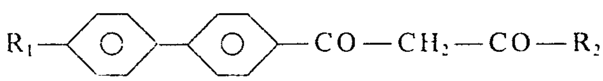

(Ib)

où

$R_1$ représente un alcoyle, un perfluoroalcoyle ou un alcoxy en $C_1$ à $C_{18}$ et

$R_2$ représente un alcoyle ou un perfluoroalcoyle en $C_1$ à $C_{12}$.

4. Dicétones selon la revendication 1 de formule (Ic)

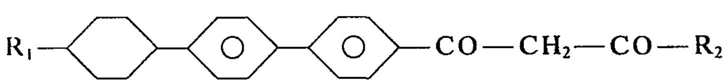

(Ic)

où

$R_1$ représente un alcoyle en $C_1$ à $C_{18}$ et

$R_2$ représente un alcoyle ou un perfluoroalcoyle en $C_1$ à $C_{12}$.

5. Procédé de préparation des dicétones de formule (I)

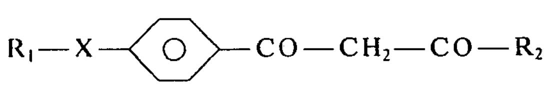

(I)

où

$R_1$ représente un alcoyle, un perfluoroalcoyle ou un alcoxy en $C_1$ à $C_{18}$,

$R_2$ représente un alcoyle ou un perfluoroalcoyle en $C_1$ à $C_{12}$ et

X représente un 1,4-trans-cyclohexylène, un 1,4-phénylène ou un 4,4'-cyclohexylphényle, caractérisé en ce qu'on fait réagir une acétophénone de formule (II)

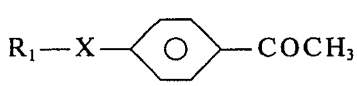

(II)

où $R_1$ a la signification donnée ci-dessus, en présence d'un agent de condensation basique avec un ester d'acide carboxylique de formule (III)

$R_2—COO—Z$ (III)

où

Z est un groupe alcoyle inférieur et

$R_2$ a la signification donnée ci-dessus,

puis en ce qu'on hydrolyse le sel de la forme énol de la dicétone (I) apparu pour donner celle-ci.

6. Application d'une dicétone de formule (I)

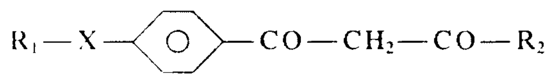 (I)

ou

$R_1$ représente un alcoyle, un perfluoroalcoyle ou un alcoxy en $C_1$ à $C_{18}$,

$R_2$ représente un alcoyle ou un perfluoroalcoyle en $C_1$ à $C_{12}$ et

X représente un 1,4-trans-cyclohexylène, un 1,4-phénylène ou un 4,4'-cyclohexylphényle,

comme composant d'un diélectrique à cristaux liquides.

7. Diélectrique à cristaux liquides, caractérisé en ce qu'il contient au moins une dicétone de formule (I)

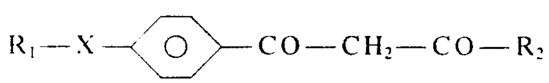 (I)

où

$R_1$ représente un alcoyle, un perfluoroalcoyle ou un alcoxy en $C_1$ à $C_{18}$,

$R_2$ représente un alcoyle ou un perfluoroalcoyle en $C_1$ à $C_{12}$ et

X représente un 1,4-trans-cyclohexylène, 1,4-phénylène ou 5,4'-cyclohexylphényle.

8. Elément d'affichage électro-optique à base d'une cellule à cristaux liquides, caractérisé en ce qu'il contient un diélectrique à cristaux liquides selon la revendication 7.

**Revendications pour l'Etat contractant: AT**

1. Procédé de préparation des dicétones de formule (I)

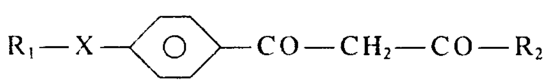 (I)

où

$R_1$ représente un alcoyle, un perfluoroalcoyle ou un alcoxy en $C_1$ à $C_{18}$,

$R_2$ représente un alcoyle ou un perfluoroalcoyle en $C_1$ à $C_{12}$ et

X représente un 1,4-trans-cyclohexylène, un 1,4-phénylène ou un 4,4'-cyclohexylphényle,

caractérisé en ce qu'on fait réagir une acétophénone de formule (II)

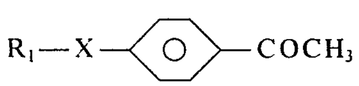 (II)

où $R_1$ a la signification donnée ci-dessus, en présence d'un agent de condensation basique, avec un ester d'acide carboxylique de formule (III)

$R_2—COO—Z$ (III)

où

Z est un groupe alcoyle inférieur et où

$R_2$ a la signification donnée ci-dessus,

puis en ce qu'on hydrolyse le sel de la forme énol de la dicétone (I) apparu pour donner celle-ci.

2. Application d'une dicétone de formule (I)

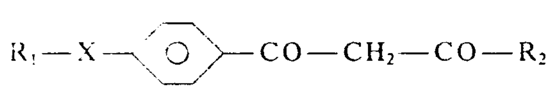 (I)

où

$R_1$ représente un alcoyle, un perfluoroalcoyle ou un alcoxy en $C_1$ à $C_{18}$,

$R_2$ représente un alcoyle ou un perfluoroalcoyle en $C_1$ à $C_{12}$ et

X représente un 1,4-trans-cyclohexylène, un 1,4-phénylène ou un 4,4'-cyclohexylphényle,

comme composant d'un diélectrique à cristaux liquides.

3. Diélectrique à cristaux liquides, caractérisé en ce qu'il contient au moins une dicétone de formule (I)

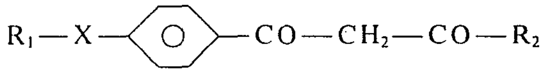

(I)

où

$R_1$ représente un alcoyle, un perfluoroalcoyle, ou un alcoxy en $C_1$ à $C_{18}$,

$R_2$ représente un alcoyle ou un perfluoroalcoyle en $C_1$ à $C_{12}$ et

X représente un 1,4-trans-cyclohexylène, un 1,4-phénylène ou un 4,4'-cyclohexylphényle.

4. Elément d'affichage électro-optique à base d'une cellule à cristaux liquides, caractérisé en ce qu'il contient un diélectrique à cristaux liquides selon la revendication 3.